# FASCICULE DE BREVET EUROPEEN

(11) **EP 0 756 863 B1**
(45) Date de publication et mention de la délivrance du brevet: **29.12.1997**
(21) Numéro de dépôt: 96401490.6
(22) Date de dépôt: 05.07.1996
(51) Int. Cl.: A61K 7/48, A61K 7/02, A61K 7/025, A61K 7/031, A61K 7/032

(54) **Procédé de préparation d'une composition comprenant un composé sensible à la chaleur et composition obtenue**
Herstellungsverfahren einer zu eine wärmeempfindliche Verbindung enthaltenden Zubereitung und die dabei enthaltene Zubereitung
Preparation process of a composition containing a heat-sensitive compound and the composition obtained thereby

(30) Priorité: 28.07.1995 FR 9509255
(43) Date de publication de la demande: 05.02.1997
(73) Titulaire: L'OREAL, 75008 Paris (FR)
(72) Inventeur: Agostini, Isabelle, 92290 Chatenay Malabry (FR)
(74) Mandataire: Dodin, Catherine

(56) Documents cités:
- EP-A- 0 530 084
- EP-A- 0 530 085

## Description

La présente invention a trait à une composition notamment cosmétique pouvant se présenter sous forme d'une pâte souple, et susceptible d'être utilisée pour le soin et/ou le maquillage des lèvres et/ou de la peau.
L'invention a également trait à un procédé de préparation de ladite composition.

Les compositions notamment cosmétiques pouvant être appliquées sur la peau ou les lèvres comme produit de maquillage ou de soin, telles que les bases pour lèvres ou les rouges à lèvres par exemple, contiennent généralement des corps gras dont des cires, des pigments et/ou charges et, éventuellement, des additifs. Il est également connu des compositions cosmétiques se présentant sous forme de pâte souple, applicable à l'aide d'un pinceau, par exemple. Ces compositions ne contiennent généralement pas ou peu de cires, notamment en une faible quantité, ce qui permet de les prélever et de les appliquer aisément. Il est également connu des compositions dans lesquelles au moins une partie des huiles est remplacée par des huiles volatiles. Ceci permet d'améliorer, entre autre, la tenue du film par concentration des constituants par évaporation des composés volatils après application.

Selon l'art antérieur, les compositions sont généralement préparées par mélange à chaud, à une température généralement de l'ordre de 95-100°C, des différents constituants à l'exception des huiles volatiles, puis introduction à une température du mélange moins élevée, mais de l'ordre de 75-80°C quand même, desdites huiles volatiles. Il n'est en effet pas possible d'introduire lesdites huiles volatiles dans un mélange 'froid', sans observer de cristallisation et de prise en masse des cires.
Le procédé selon l'état de la technique implique, d'une part, une perte par évaporation d'une partie desdites huiles volatiles lors de la préparation de la composition, d'où la nécessité d'augmenter par anticipation la quantité à ajouter afin d'obtenir l'effet final souhaité. D'autre part, la manipulation à une température élevée d'huiles volatiles de faible point éclair implique des conditions de sécurité relativement importantes.

D'autre part, on connaît par le document EP305756 un système permettant de délivrer de manière continue ou discontinue, un actif sur un support. Ce système est constitué d'un mélange d'un composé sensible à la pression et d'un actif; on applique ledit système sur le support en le chauffant à 40-80°C. Le mélange peut notamment être effectué par extrusion, mélange direct ou à l'aide de rouleaux, de couteaux ou par atomisation.

La présente invention a pour but de proposer un nouveau procédé palliant les inconvénients de l'art antérieur et permettant l'introduction notamment 'à froid' de composés par exemple volatils et de faible point éclair dans une composition comprenant des constituants de température de fusion élevée, et ce tout en obtenant en final, une composition homogène.

Un objet de la présente invention est donc un procédé de préparation d'une composition cosmétique, pharmaceutique ou hygiénique, se présentant sous la forme d'une pâte souple de viscosité dynamique à 25°C comprise entre 3 et 35 Pa.s, et comprenant au moins un composé sensible à la chaleur, dans lequel on incorpore ledit composé sensible à la chaleur dans la composition à l'aide d'un extrudeur.
Un autre objet de l'invention est une composition susceptible d'être obtenue à l'aide du procédé précédent, se présentant sous forme d'une pâte souple de viscosité dynamique à 25°C comprise entre 3 et 35 Pa.s et comprenant au moins un composé sensible à la chaleur et au moins une cire.

La présente invention trouve en particulier une application dans les domaines cosmétique, pharmaceutique et hygiénique, lors de la préparation de compositions homogènes comprenant d'une part des composés ayant une température de fusion T, et d'autre part des composés qu'il n'est pas possible de chauffer jusqu'à cette température T, appelés composés sensibles à la température T, par exemple parce qu'ils s'y dénaturent ou s'y volatilisent.
Ainsi, l'invention peut trouver une application dans la fabrication de produits de maquillage et notamment dans la fabrication de produits qui contiennent des cires et des composés volatils, tels que des rouges à lèvres.

Par température 'sensible' du composé, on entend la température à laquelle, par exemple, il se volatilise ou il se dénature, c'est-à-dire la température au-delà de laquelle il n'est pas possible de le chauffer sans engendrer de détérioration, de changement d'état ou de modification de sa composition, de sa nature chimique, de sa structure, etc.

Ainsi, l'invention permet de préparer une composition comprenant des cires, composés à température de fusion 'élevée', et des composés sensibles à la chaleur, notamment des composés volatils et/ou de faible point éclair.

Par composé volatil, on entend dans la présente description tout composé susceptible de s'évaporer au contact de la peau.
Par composé volatil de faible point éclair, on entend dans la présente description tout composé dont le point éclair est inférieur à environ 100°C, et notamment de point éclair compris entre 30 et 85°C, c'est-à-dire susceptible de s'enflammer, en présence d'une étincelle, à une température usuelle utilisée lors de la préparation de compositions comprenant des cires.

Parmi les composés volatils de faible point éclair, on peut citer certaines huiles hydrocarbonées ou siliconées, linéaires ou cycliques, telles que la cyclotétradiméthylsiloxane (point éclair 55°C), la cyclopentadiméthylsiloxane (point éclair environ 77°C), la cyclohexadiméthylsiloxane (point éclair 76°C), le méthylhexyldiméthylsiloxane (point éclair 79°C), les isoparaffines (point éclair 56°C). Ces huiles peuvent être utilisées seules ou en mélange.

Les cires pouvant être présentes dans la composition ont de préférence un point de fusion supérieur à 55°C et un indice de pénétration de l'aiguille à 25°C de préférence compris entre 3 et 40. Parmi les cires envisageables, seules ou en mélange, on peut citer les cires animales, végétales, minérales et synthétiques, telles que la cire d'abeilles; la cire de Carnauba, de Candellila, d'Ourrury, du Japon, les cires de fibres de lièges ou de canne à sucre; les cires de paraffine, de lignite; les cires microcristallines; les ozokérites; les cires de polyéthylène et les cires obtenues par synthèse de Fischer-Tropsch; les cires siliconées.
On a constaté que bien que la composition selon l'invention comprenne une quantité importante de cires, de préférence de l'ordre de 12-60% en poids, il est possible d'y introduire des composés volatils à froid, sans observer d'amorce de cristallisation desdites cires. On peut ainsi obtenir une pâte souple homogène et stable.

La composition selon l'invention peut en outre comprendre des huiles, volatiles de haut point éclair, notamment dont le point éclair est supérieur à 100°C, et/ou non volatiles, siliconées et/ou hydrocarbonées, seules ou en mélange, ainsi que tout autre corps gras usuel dans le domaine cosmétique.

On peut par exemple citer les huiles de silicone telles que les polydiméthylsiloxanes, les huiles siliconées phénylées notamment les polyphénylméthylsiloxanes ou les phényltriméthicones, ou leurs mélanges, et en particulier les huiles siliconées phénylées répondant à la formule suivante : 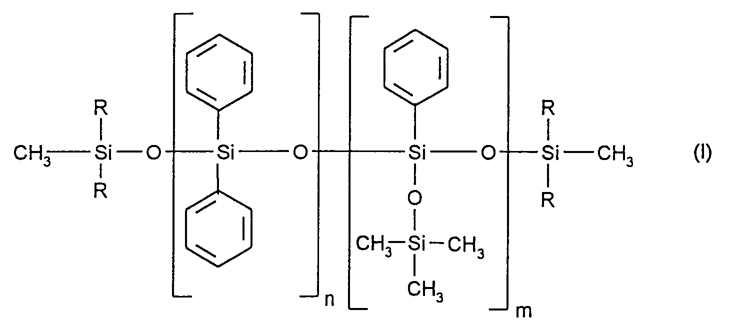 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.
De préférence, R est un radical méthyle, éthyle, propyle, isopropyle, décyle, dodécyle ou octadécyle, ou encore un radical phényle, tolyle, benzyle ou phénéthyle.
Parmi ces huiles phénylées, on peut citer l'huile Belsil PDM1000 de Wacker, les huiles DC556 ou SF558 de Dow Corning, l'huile Abil AV8853 de Goldschmidt ou l'huile Silbione 70633V30 de Rhône Poulenc.

On peut également citer les huiles végétales, minérales, animales et/ou synthétiques, telles que l'huile de paraffine, de vaseline, le perhydrosqualène, l'huile d'arara, d'amande douce, de calophyllum, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras.

La composition peut également comprendre notamment des pigments et/ou des nacres et/ou des charges habituellement utilisés dans les compositions cosmétiques. Les pigments peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.
Les nacres peuvent être choisies parmi le mica recouvert d'oxyde de titane, d'oxyde de fer, de pigment naturel ou d'oxychlorure de bismuth ainsi que le mica titane coloré.
Les charges peuvent être minérales ou de synthèse, lamellaires ou non lamellaires. On peut citer le talc, le mica, la silice, le kaolin, les poudres de Nylon et de polyéthylène, le Téflon, l'amidon, le micatitane, la nacre naturelle, le nitrure de bore, les microsphères telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des sphingocéryls, des filtres solaires, des tensioactifs, des polymères liposolubles comme les polymères hydrocarbonés et notamment le polybutène, les polyalkylènes, les polyacrylates et les polymères siliconés compatibles avec les corps gras.

Les proportions relatives de chacun des constituants de la composition selon l'invention peuvent être déterminées par l'homme du métier sur base de ses connaissances générales et sur base de l'art antérieur, selon la destination de la composition, étant entendu que ledit homme du métier veillera à choisir ces constituants, et/ou leur quantité, de manière telle que les propriétés avantageuses de la composition selon l'invention ne soient pas, ou substantiellement pas, altérées par l'adjonction envisagée.

La composition selon la présente invention peut se présenter sous la forme d'une pâte souple dont on peut mesurer la viscosité, par opposition à la structure solide d'un bâton, ou stick, dont on ne peut pas mesurer la viscosité. Ladite viscosité dynamique à 25°C est généralement comprise entre 3 et 35 Pa.s, mesurée avec un viscosimètre rotatif CONTRAVES TV équipé d'un mobile "MS-r4" à la fréquence de 60 Hz.
Les compositions selon l'invention peuvent ainsi se présenter sous la forme d'un produit de maquillage de la peau et/ou des lèvres. Notamment, elles peuvent se présenter sous la forme d'un fond de teint, d'un fard à joues ou à paupières, ou d'un rouge à lèvres. Lorsqu'elles contiennent des actifs, elles peuvent se présenter sous la forme d'une base de soin pour les lèvres. Elles peuvent également être utilisées en tant que base fixante à appliquer sur un rouge à lèvres classique. La base forme alors un film protecteur sur le film de rouge, en limite le transfert et la migration, et permet d'augmenter ainsi sa tenue.

Les compositions selon l'invention peuvent également se présenter sous la forme d'un produit de soin de la peau, d'un produit hygiénique ou pharmaceutique ou encore d'un produit solaire ou autobronzant.

Afin de préparer la composition selon l'invention, on peut préparer tout d'abord un prémélange comprenant au moins une partie des différents constituants de la composition, dont les composés ayant une température de fusion T qui peut être élevée, tels que les cires, on chauffe ce prémélange à une température à laquelle il fond, on ajoute le cas échéant tout ou partie des autres constituants, en une ou plusieurs fois, puis l'on ajoute à une température inférieure à sa température 'sensible', le ou les composé(s) sensible(s) à la température T, ainsi qu'éventuellement le reste des constituants, tout en maintenant un certain malaxage du mélange à l'aide d'un extrudeur.
Enfin, on malaxe dans un extrudeur le mélange obtenu pendant au moins une partie de son refroidissement jusqu'à la température recherchée, la température ambiante généralement.

Lorsque le composé sensible à la chaleur est une huile volatile que l'on souhaite incorporer dans une composition comprenant des cires, on l'introduit de préférence dans le mélange à une température inférieure à 45°C, voire à température ambiante.

De préférence, l'opération de mélange est également effectuée dans un extrudeur.
Les opérations de chauffage, de mélange, de malaxage et de refroidissement peuvent être effectuées en totalité dans un extrudeur ou dans plusieurs extrudeurs, qui seront alors disposés à la suite les uns des autres.
Toutefois, on utilise de préférence un extrudeur bi-vis unique pour effectuer la totalité du procédé.
On a constaté que la composition obtenue après extrusion présente une douceur particulière, et procure une certaine sensation de glissant lorsqu'elle est appliquée sur la peau, tout en évitant l'aspect et la sensation de gras huileux.

Les conditions dans lesquelles l'extrusion peut être effectuée sont décrites dans la demande de brevet FR94-00756 dont le contenu est incorporé à la présente demande par référence.

L'invention est illustrée plus en détail dans les exemples suivants.

### Exemple 1

On prépare un rouge à lèvres sous forme de pâte souple, ayant la composition suivante:

| | |
|---|---|
| . cyclopentadiméthylsiloxane | 40 g |
| . polyphénylméthylsiloxane (DC556 Fluid de Dow Corning) | 20 g |
| . cires (silicone et polyéthylène) | 25 g |
| . pigments et charges | 15 g |

On introduit ces différents ingrédients, à l'exception de l'huile volatile, dans un extrudeur bi-vis, à une température d'environ 75-95°C en entrée.
On introduit l'huile volatile dans l'extrudeur en fin d'extrusion, à une température de l'ordre de 20-25°C.
On obtient en sortie une pâte souple de viscosité égale à 27 Pa.s, se présentant sous forme d'une seule phase stable et homogène, et pouvant être prélevée à l'aide d'un pinceau pour son application.
Cette composition permet l'obtention d'un film homogène, facilement applicable et s'étalant facilement et uniformément. Le film obtenu présente également une texture légère et reste confortable à porter tout au long de la journée.

Si l'on souhaitait préparer cette même composition par un procédé classique, il serait nécessaire de chauffer les différents ingrédients, sauf l'huile volatile, à 95-100°C pour obtenir la fusion des cires, puis de préchauffer l'huile volatile à environ 65°C et de l'introduire dans le mélange à 75-80°C afin d'éviter la reprise en masse desdites cires.

## Revendications

1. Procédé de préparation d'une composition cosmétique, pharmaceutique ou hygiénique, se présentant sous la forme d'une pâte souple de viscosité dynamique à 25°C comprise entre 3 et 35 Pa.s, mesurée avec un viscosimètre rotatif CONTRAVES TV équipé d'un mobile "MS-r4" à la fréquence de 60 Hz, et comprenant au moins un composé sensible à la chaleur, dans lequel on incorpore ledit composé sensible à la chaleur dans la composition à l'aide d'un extrudeur.

2. Procédé selon la revendication 1 pour la préparation d'une composition comprenant d'une part des composés ayant une température de fusion T et d'autre part des composés sensibles à la température T, dans lequel . on prépare un prémélange comprenant au moins une partie des différents constituants de la composition, dont au moins les composés ayant une température de fusion T,
. on chauffe ce prémélange à une température à laquelle il fond, puis
. on ajoute les composés sensibles, à une température inférieure à leur température 'sensible' et tout en maintenant un certain malaxage du mélange,
. et l'on continue de malaxer le mélange obtenu pendant une partie de son refroidissement jusqu'à la température recherchée,
. les opérations de malaxage étant effectuées dans au moins un extrudeur.

3. Procédé selon l'une des revendications précédentes, dans lequel les composés sensibles sont choisis parmi les composés volatils et/ou de faible point éclair.

4. Procédé selon l'une des revendications précédentes, dans lequel les composés sensibles sont choisis parmi la cyclotétradiméthylsiloxane, la cyclopentadiméthylsiloxane, la cyclohexadiméthylsiloxane, le méthylhexyldiméthylsiloxane, les isoparaffines et leur mélange.

5. Procédé selon l'une des revendication 2 à 4, dans lequel les composés ayant une température de fusion T sont des cires.

6. Procédé selon l'une des revendications 2 à 5 dans lequel les composés ayant une température de fusion T sont des cires choisies parmi les cires animales, végétales, minérales et synthétiques, telles que la cire d'abeilles; la cire de Carnauba, de Candellila, d'Ourrury, du Japon, les cires de fibres de lièges ou de canne à sucre; les cires de paraffine, de lignite; les cires microcristallines; les ozokérites; les cires de polyéthylène et les cires obtenues par synthèse de Fischer-Tropsch; les cires siliconées.

7. Procédé selon l'une des revendications 5 à 6 dans lequel les cires sont présentes dans la composition à raison de 12-60% en poids.

8. Procédé selon l'une des revendications précédentes pour la préparation d'un produit de maquillage de la peau et/ou des lèvres tel qu'un fond de teint, un fard à joues ou à paupières, un rouge à lèvres; d'une base de soin pour les lèvres; base fixante pour rouge à lèvres; un produit de soin de la peau; un produit hygiénique ou pharmaceutique; un produit solaire ou autobronzant.

9. Procédé selon l'une des revendications précédentes, dans lequel l'opération de mélange est également effectuée dans un extrudeur.

10. Procédé selon l'une des revendications précédentes, dans lequel les opérations de chauffage, de mélange, de malaxage et de refroidissement sont effectuées en totalité dans plusieurs extrudeurs disposés à la suite les uns des autres.

11. Procédé selon l'une des revendications 1 à 9 dans lequel les opérations de chauffage, de mélange, de malaxage et de refroidissement sont effectuées en totalité dans un extrudeur bi-vis unique.

12. Composition susceptible d'être obtenue à l'aide du procédé selon l'une des revendications 1 à 11, se présentant sous forme d'une pâte souple ayant une viscosité dynamique à 25°C comprise entre 3 et 35 Pa.s, mesurée avec un viscosimètre rotatif CONTRAVES TV équipé d'un mobile "MS-r4" à la fréquence de 60 Hz, et comprenant au moins un composé sensible à la chaleur et au moins une cire.

13. Composition selon la revendication 12, dans laquelle la cire est présente dans la composition à raison de 12-60% en poids.

14. Composition selon l'une des revendications 12 à 13, dans laquelle le composé sensible à la chaleur est choisi parmi la cyclotétradiméthylsiloxane, la cyclopentadiméthylsiloxane, la cyclohexadiméthylsiloxane, le méthylhexyldiméthylsiloxane, les isoparaffines et leur mélange.

15. Composition selon l'une des revendication 12 à 14, dans laquelle les cires sont choisies parmi les cires animales, végétales, minérales et synthétiques, telles que la cire d'abeilles; la cire de Carnauba, de Candellila, d'Ourrury, du Japon, les cires de fibres de lièges ou de canne à sucre; les cires de paraffine, de lignite; les cires microcristallines; les ozokérites; les cires de polyéthylène et les cires obtenues par synthèse de Fischer-Tropsch; les cires siliconées.

16. Composition selon l'une des revendications 12 à 15 se présentant sous la forme d'un produit de maquillage de la peau et/ou des lèvres tel qu'un fond de teint, un fard à joues ou à paupières, un rouge à lèvres; d'une base de soin pour les lèvres; base fixante pour rouge à lèvres; un produit de soin de la peau; un produit hygiénique ou pharmaceutique; un produit solaire ou autobronzant.

## Claims

1. Process for the preparation of a cosmetic, pharmaceutical or hygiene composition which is in the form of a supple paste with a dynamic viscosity at 25°C of between 3 and 35 Pa s, measured with a Contraves TV rotary viscometer fitted with an "MS-r4" rotor, at a frequency of 60 Hz, and which includes at least one heat-sensitive compound, in which the said heat-sensitive compound is incorporated into the composition with the aid of an extruder.

2. Process according to Claim 1 for the preparation of a composition including, on the one hand, compounds which have a melting temperature T and, on the other hand, compounds which are sensitive at the temperature T, in which
- a premix is prepared including at least some of the various constituents of the composition, including at least the compounds which have a melting temperature T,
- this premix is heated to a temperature at which it melts, then
- the sensitive compounds are added at a temperature which is lower than their "sensitive" temperature and while a certain amount of blending of the mixture is maintained,
- and blending of the mixture obtained is continued during a part of its cooling to the required temperature,
- the blending operations being performed in at least one extruder.

3. Process according to either of the preceding claims, in which the sensitive compounds are chosen from compounds which are volatile and/or of low flash point.

4. Process according to one of the preceding claims, in which the sensitive compounds are chosen from cyclotetradimethylsiloxane, cyclopentadimethylsiloxane, cyclohexadimethylsiloxane, methylhexyldimethylsiloxane, isoparaffins and a mixture thereof.

5. Process according to one of Claims 2 to 4, in which the compounds which have a melting temperature T are waxes.

6. Process according to one of Claims 2 to 5, in which the compounds which have a melting temperature T are waxes chosen from animal, plant, mineral and synthetic waxes such as beeswax, carnauba, candelilla, ouricurry and Japan wax, cork fibre or sugarcane waxes, paraffin and lignite waxes, microcrystalline waxes, ozokerites, polyethylene waxes and the waxes obtained by Fischer-Tropsch synthesis, and silicone waxes.

7. Process according to either of Claims 5 and 6, in which the waxes are present in the composition in a proportion of 12-60 % by weight.

8. Process according to one of the preceding claims, for the preparation of a make-up product for the skin and/or the lips, such as a foundation, a blusher, an eyeshadow, a coloured lip make-up, a care base for the lips, a fixing base for coloured lip make-up, a skincare product, a hygiene or pharmaceutical product or an antisun or self-tanning product.

9. Process according to one of the preceding claims, in which the mixing operation is also performed in an extruder.

10. Process according to one of the preceding claims, in which the heating, mixing, blending and cooling operations are performed entirely in several extruders arranged one after the other.

11. Process according to one of Claims 1 to 9, in which the heating, mixing, blending and cooling operations are performed entirely in a single twin-screw extruder.

12. Composition which may be obtained using the process according to one of Claims 1 to 11, in the form of a supple paste having a dynamic viscosity at 25°C of between 3 and 35 Pa s, measured with a Contraves TV rotary viscometer fitted with an "MS-r4" rotor, at a frequency of 60 Hz, and comprising at least one heat-sensitive compound and at least one wax.

13. Composition according to Claim 12, in which the wax is present in the composition in a proportion of 12-60 % by weight.

14. Composition according to either of Claims 12 and 13, in which the heat-sensitive compound is chosen from cyclotetradimethylsiloxane, cyclopentadimethylsiloxane, cyclohexadimethylsiloxane, methylhexyldimethylsiloxane, isoparaffins and a mixture thereof.

15. Composition according to one of Claims 12 to 14, in which the waxes are chosen from animal, plant, mineral and synthetic waxes such as beeswax, carnauba, candelilla, ouricurry and Japan wax, cork fibre or sugarcane waxes, paraffin and lignite waxes, microcrystalline waxes, ozokerites, polyethylene waxes and the waxes obtained by Fischer-Tropsch synthesis, and silicone waxes.

16. Composition according to one of Claims 12 to 15, which is in the form of a make-up product for the skin and/or the lips such as a foundation, a blusher, an eyeshadow, a coloured lip make-up, a care base for the lips, a fixing base for coloured lip make-up, a skincare product, a hygiene or pharmaceutical product or an antisun or self-tanning product.

## Patentansprüche

1. Verfahren zur Herstellung einer kosmetischen, pharmazeutischen oder hygienischen Zusammensetzung in Form einer weichen Paste mit einer dynamischen Viskosität von 3 bis 35 Pa·s bei 25°C, die mit einem Rotationsviskosimeter CONTRAVES TV bestimmt wurde, das mit einem beweglichen "MS-r4" einer Frequenz von 60 Hz ausgestattet war, die mindestens eine wärmeempfindliche Verbindung enthält, wobei die wärmeempfindliche Verbindung in die Zusammensetzung mit einem Extruder eingearbeitet wird.

2. Verfahren nach Anspruch 1 zur Herstellung einer Zusammensetzung, die einerseits Verbindungen mit einem Schmelzpunkt T und andererseits Verbindungen enthält, die bei der Temperatur T empfindlich sind, wobei
- ein Vorgemisch hergestellt wird, das mindestens einen Teil der verschiedenen Bestandteile der Zusammensetzung umfaßt, darunter mindestens die Verbindungen mit dem Schmelzpunkt T
- das Vorgemisch auf eine Temperatur erwärmt wird, bei der es schmilzt,
- dann die empfindlichen Verbindungen bei einer Temperatur zugegeben werden, die unter der "empfindlichen" Temperatur liegt, wobei ein bestimmtes Kneten des Gemisches aufrechterhalten wird, und
- das Kneten des erhaltenen Gemisches während eines Teils der Abkühlung auf die gewünschte Temperatur fortgesetzt wird,
- wobei das Kneten mit mindestens einem Extruder durchgeführt wird.

3. Verfahren nach einem der vorhergehenden Ansprüche, wobei die empfindlichen Verbindungen unter den flüchtigen Verbindungen und/oder Verbindungen mit einem niedrigen Flammpunkt ausgewählt sind.

4. Verfahren nach einem der vorhergehenden Ansprüche, worin die empfindlichen Verbindungen unter Cyclotetradimethylsiloxan, Cyclopentadimethylsiloxan, Cyclohexadimethylsiloxan, Methylhexyldimethylsiloxan, Isoparaffinen und deren Gemischen ausgewählt sind.

5. Verfahren nach einem der Ansprüche 2 bis 4, worin die Verbindungen mit einem Schmelzpunkt T Wachse sind.

6. Verfahren nach einem der Ansprüche 2 bis 5, worin die Verbindungen mit einem Schmelzpunkt T Wachse sind, die unter tierischen, pflanzlichen, mineralischen und synthetischen Wachsen ausgewählt sind, wie Bienenwachs, Carnaubawachs, Candellilawachs, Ouricurywachs, Japanwachs, Korkwachs oder Zuckerrohrwachs, Paraffinwachsen, Lignitwachs, mikrokristallinen Wachsen, Ozokeriten, Polyethylenwachsen, Wachsen, die durch Fischer-Tropsch-Synthese erhalten wurden, und Siliconwachsen.

7. Verfahren nach einem der Ansprüche 5 oder 6, worin die Wachse in der Zusammensetzung in einem Anteil von 12 bis 60 Gew.-% vorliegen.

8. Verfahren nach einem der vorhergehenden Ansprüche zur Herstellung eines Schminkproduktes für die Haut und/oder die Lippen, wie ein Make-up, Rouge oder Lidschatten, Lippenstift, Pflegegrundstoffe für die Lippen, Fixierungsgrundstoffe für Lippenstifte, Pflegeprodukte für die Haut, hygienische oder pharmazeutische Produkte und Sonnenschutz- oder Selbstbräunungsprodukte.

9. Verfahren nach einem der vorhergehenden Ansprüche, worin das Vermischen ebenfalls in einem Extruder durchgeführt wird.

10. Verfahren nach einem der vorhergehenden Ansprüche, worin die Arbeitsschritte des Erwärmens, des Vermischens, des Knetens und des Abkühlens insgesamt in mehreren Extrudern durchgeführt werden, die aufeinanderfolgend angeordnet sind.

11. Verfahren nach einem der Ansprüche 1 bis 9, worin die Arbeitsschritte des Erwärmens, des Vermischens, des Verknetens und des Abkühlens insgesamt in einem einzigen Zweischneckenextruder durchgeführt werden.

12. Zusammensetzung, die nach dem Verfahren nach einem der Ansprüche 1 bis 11 erhalten werden kann in Form einer weichen Paste mit einer dynamischen Viskosität von 3 bis 35 Pa·s bei 25°C vorliegt, welche mit einem Rotationsviskometer CONTRAVES TV bestimmt wurde, das mit einem beweglichen "MS-r4" einer Frequenz von 60 Hz ausgestattet ist, und mindestens eine wärmeempfindliche Verbindung und mindestens ein Wachs enthält.

13. Zusammensetzung nach Anspruch 12, worin das Wachs in einem Anteil von 12 bis 60 Gew.-% in der Zusammensetzung vorliegt.

14. Zusammensetzung nach einem der Ansprüche 12 bis 13, worin die wärmeempfindliche Verbindung unter Cyclotetradimethylsiloxan, Cyclopentadimethylsiloxan, Cyclohexadimethylsiloxan, Methylhexyldimethylsiloxan, Isoparaffinen und deren Gemischen ausgewählt ist.

15. Zusammensetzung nach einem der Ansprüche 12 bis 14, worin die Wachse unter tierischen, pflanzlichen, mineralischen und synthetischen Wachsen ausgewählt sind, wie Bienenwachs, Carnaubawachs, Candellilawachs, Ouricurywachs, Japanwachs, Korkwachs oder Zuckerrohrwachs, Paraffinwachsen, Lignitwachs, mikrokristallinen Wachsen, Ozokeriten, Polyethylenwachsen, Wachsen, die durch Fischer-Tropsch-Synthese erhalten wurden, und Siliconwachsen.

16. Zusammensetzung nach einem der Ansprüche 12 bis 15 in Form eines Schminkproduktes für die Haut und/oder die Lippen, wie Make-up, Rouge, Lidschatten, Lippenstift, Pflegegrundstoffe für die Lippen, Fixierungsgrundstoffe für Lippenstifte, Pflegeprodukte für die Haut, hygienische oder pharmazeutische Produkte und Sonnenschutz- oder Selbstbräunungsprodukte.
